(19) Europäisches Patentamt
European Patent Office
Office européen des brevets

(11) Publication number: **0 486 958 A1**

(12) **EUROPEAN PATENT APPLICATION**

(21) Application number: **91119503.0**

(22) Date of filing: **15.11.91**

(51) Int. Cl.5: **C07D 487/04**, A61K 31/40, //(C07D487/04,209:00,209:00)

(30) Priority: **20.11.90 IT 2211190**

(43) Date of publication of application: **27.05.92 Bulletin 92/22**

(84) Designated Contracting States: **AT BE CH DE DK ES FR GB GR IT LI LU NL SE**

(71) Applicant: **MEDIOLANUM FARMACEUTICI S.p.a.**
**Via S. Giuseppe Cottolengo, 31**
**I-20143 Milan(IT)**

(72) Inventor: **Pagella, Pier Giuseppe**
**Isola S. Antonio**
**I-15050 Frazione Catraglia(Allessandria)(IT)**
Inventor: **Brufani, Mario**
**Via Aldo Moro, 28**
**I-00040 Castelgandolfo (Prov. Roma)(IT)**
Inventor: **Marta, Maurizio (UNIV.CATT.DEL SACRO CUORE)**
**Largo F.Vito, 1**
**I-00168 Roma(IT)**
Inventor: **Pomponi, Massimo (UNIV.CATT.DEL SACRO CUORE)**
**Largo F.Vito, 1**
**I-00168 Roma(IT)**

(74) Representative: **Gervasi, Gemma et al**
**NOTARBARTOLO & GERVASI Srl Viale Bianca Maria 33**
**I-20122 Milan(IT)**

(54) **Physostigmine derivatives.**

(57) Physostigmine derivatives are described of the general formula:

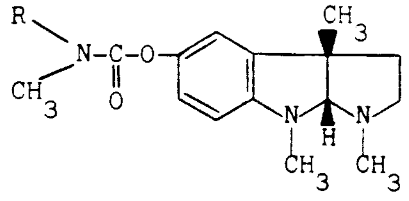

(I)

in which R is a linear or branched alkyl with three to nine C atoms, or benzyl, and their slats with pharmaceutically acceptable acids, which are particularly useful for the preparation of pharmaceutical compositions having a acetylcholinesterase inhibiting function.

EP 0 486 958 A1

Technical field

The present invention relates to physostigmine derivatives and to their salts with pharmaceutically acceptable acids. More particularly, the present invention relates to certain particular physostigmine derivatives of the general formula:

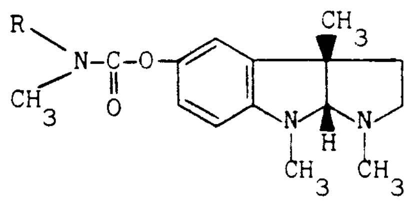

(I)

wherein R is a linear or branched alkyl with 3 to 9 carbon atoms or benzyl, and to their salts with pharceutically acceptable acids.

The thicker bonds in formula (I) and in the other structural formulas appearing in the present text in correspondence with the methyl groups and hydrogen atoms bound in positions 3a and 8a of the three rings indicated that the methyl groups and the hydrogen atom are on a same side of the plane of said rings. The invention also relates to the use of physostigmine derivatives of formula (I) and of their salts with pharmaceutically acceptable acids for the preparation of pharmaceutical compositions having acetylcholinesterase inhibiting function.

Prior Art

The anticholinesterase function of physostigmine has been known for a long time. It is also known, on the other hand, that in Alzheimer's dementia a considerable decrease of the acetylcholine cerebral concentration takes place and that, therefore, the use of drugs apt to increase that concentration is useful.

Physostigmine, although being suitable to this end, has a high toxicity, a short activity and peripheral damaging effects, particularly on the digestige system.

Physostigmine derivatives having lower toxicity are also known and are described e.g. in the European Patent Application EP-A-154.864.

The applicant has described salts of physostigmine derivatives, having an unsubstituted hydrogen atom on the nitrogen of the carbamic group, in the European Patent Application EP-A-298.202. Said salts show a high water solubility, a high stability to light and air, a low toxicity and a protracted activity.

Other physostigmine derivatives are described in the European Patent Application EP-A-253,372, having in particular the nitrogen in the carbamic group substituted in such a way as to form a hetero-atom in a heterocyclic nucleus with five or six atoms, said derivatives having analgesic and mnemonics improving properties.

Technical problem

The particular activity of physostigmine derivatives in the field of cerebral affections, leads to a continuous search for new compounds, in the hope of succeeding in the synthesis of new derivatives possessing both a high anticholinergic activity and low toxicity.

Detailed description of the invention

We now have surprisingly found that physostigmine derivatives of formula (I) and their salts with pharmaceutically acceptable acids, in accordance with the present invention, in which derivatives one of the two substituents on the carbamic acid nitrogen is a methyl group and the other is a linear or branched alkyl with 3 to 9 carbon atoms or benzyl, show a reduced toxicity and a higher anticholinergic activity with respect to similar compounds having a hydrogen atom, rather than a methyl group, bound to the nitrogen in the carbamic group.

This characteristic of the physostigmine derivatives of formula (I) is very surprising in view of the fact that similar derivatives having two methyl or two ethyl groups bound to the carbamic group nitrogen,

although having a slightly lower toxicity in comparison with other known physostigmine derivatives, have a very weak acetylcholinesterase inhibiting activity and were not considered sufficiently interesting for further pharmacological investigations.

Experimental data on the low inhibiting activity of diethylphysostigmine were published in Biochem. Acta 47 (1988) 3, pages 285-88.

Among all the organic or inorganic pharmaceutically acceptable acids which may be employed to form the salts of formula (I) derivatives according to the invention, the ones preferred are the organic acids, and among these particularly preferred are citric acid, tartaric acid and maleic acid.

The process for preparing the derivatives of formula according to the present invention is the following:

a) Physostigmine is reacted, at room temperature and in a nitrogen atmosphere, with sodium methoxide and an aliphatic alchool having 1 to 3 carbon atoms in a low polarity organic solvent, transforming it into eseroline (II)

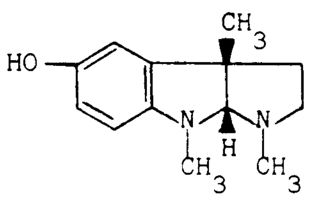

(II)

b) the alcohol is removed by distillation and the eseroline solution is reacted with a solution in the same solvent of a carbamyl chloride of general formula R-N($CH_3$)-CO-Cl (III) in which R is a linear or branched alkyl with 3 to 9 C atoms or benzyl, to obtain the corresponding derivative of formula (I).

c) At the end, the reaction mixture is treated with a solution in an organic solvent of the acid with which it is intended to form the physostigmine derivative salt, or the reaction mixture is washed with cold water and the crude derivative is purified by extraction with apolar solvents.

Among the organic solvents to be employed in the process according to the invention, one can mention dioxane, ethylene glycol, benzene, ethyl ether, di-isopropyl ether, toluene, xylene, petroleum ether.

The N-methyl,N-alkyl-carbamyl chloride solution is slowly added to the eseroline solution in an amount sufficient to reach a 1:1 to 1:1.4 ratio between the two reagents. At the end of the reaction, which takes 2 to 7 hours, the mixture is washed with cold water, using each time a small amount of water and operating rapidly to avoid hydrolysis of the carbamate. The crude products are then purified by extraction with apolar solvents, preferably with petroleum ether or n-heptane.

According to another embodiment of the process according to the invention, the salt of the physostigmine derivative of formula (I) is prepared by dissolving the derivative in an organic solvent, selected from the groups consisting of ethyl ether, ethanol, isopropanol, di-isopropyl ether or their mixtures, preferably isopropyl ether or isopropyl alcohol or their mixtures, and reacting the thus obtained solution with the desired acid in ethanol, methanol, isopropyl alcohol, ethyl ether, preferably in isopropyl alcohol.

The reagent concentration to obtain the salts of the physostigmine derivative of formula (I) are typically comprised between 50 and 250 ml solvent per g of physostigmine derivative, and between 50 and 350 ml solvent per g of acid. The obtained salt is purified with the usual methods of solvent extraction, chromatography and crystallization.

Thus the physostigmine salts of formula (I) are obtained in high yields, in solid, divided, non-hygroscopic form, with high purity and high stability to light and air.

The above described process is particularly useful when employing organic acids, such a tartaric, citric or maleic acid.

The carbamyl chlorides of formula R-N($CH_3$)-COCl(III), are prepared by reacting the selected secondary amine with tri-phosgene, in a hydrocarbon solvent in the presence of a nucleophilic catalyst, as described in Angew. Chem. Int. Ed. Engl. - 26- (1987) - 894/5. The preferred solvent is benzene and the preferred catalyst pyridine.

The reaction takes place at room temperature and is complete in 40 to 60 hours.

Acetylcholinesterase inhibiting activity - Test "in vitro"

The acetylcholinesterase activity was determined by the method of Ellman et al. (Ellman, G.L. Courtney,

K.D. Andres, V., Featherstone, R.M. A new and rapide colorimetric determination of acetylcholinesterase activity - Biochem. Pharm., 1961, 7, 88).

The Electric Eel acetylcholinesterase, type V.S. of Sigma Chemicals was employed.

To determine $IC_{50}$, the incubation medium consisted of: Ellman reagent (5,5'-Dithiobis-2-nitrobenzoic acid) 100 µl; enzymatic solution (5,5 units/ml) 35 µl; variable concentration inhibitor 50 µl; phosphate buffer (0.1 mol/l; pH 8) 3000 µl. After 20 min. incubation, 20 µl acetylcholine (0.075 mol/l) are added, and the spectrophotomer, with kinetic software and thermostated at 25°C, was read for 3-5 min. At this point the reaction is in the stationary state. The concentrations of the products apt to inhibit by 50% ($IC_{50}$) the enzyme activity were calculated.

The results are shown in Table 1.

**Table 1: Acetylcholinesterase inhibiting activity**

| Product | $IC_{50}$ ($10^{-6}$M) |
|---|---|
| Example 2 | 1.1 |
| Example 3 | 1.9 |
| Example 4 | 3.5 |
| Example 6 | 1.0 |

The products of the examples possess a powerful "in vitro" activity in inhibiting acetylcholinesterase. Said activity is of the order of $10^{-6}$ molar.

Evaluation of the acetylcholinesterase inhibiting activity (Enzymatic dosage)

The effects of the compounds of Examples 2 and 3 on the brain acetylcholinesterase activity of male CD/SD rats evaluation were administered by gastric probe at the dose of 50 mg/kg in the form of tartrates dissolved in water. The rats were previously kept from eating for 18 hrs. One group of rats treated by or with physiological solution served as control. The rats were sacrificed by decapitation at various times after the treatment with the products under examination. On the isolated brains the acetylcholinesterase activity was determined by the Ellman et al. method employed in the "vitro" tests.

The percentage variation of the acetylcholinesterase activity in the brain of the rats treated by or with the brains of rats treated with physiological solution (control group) was calculated. Table 2 summarizes the results.

Table 2

| % Variation of acetylcholinesterase | | | | |
|---|---|---|---|---|
| Product | N° Animal | % Variation of acetylcholinesterase at time | | |
| | | 10′ | 60′ | 120′ |
| Example 3 | 1<br>2 | -60,1<br>-53,8 | -69,6<br>-70,9 | -72,0<br>-68,0 |
| Example 4 | 1<br>2 | 0<br>5,7 | -52,7<br>-47,1 | -54,5<br>-56,5 |

The two products prove to penetrate the hematoencephalic barrier, producing a powerful and long lasting brain acetylcholinesterase inhibiting action. The compound of Example 3 has a more rapid and powerfyul action than the one of Example 4.

Evaluation of acute toxicity ($LD_{50}$)

The $LD_{50}$ acute toxicity of the compounds obtained in Examples 2,3,4 and 6 has evaluated on Suiss female rats.

The compounds were administered per os and endovenously to different groups of animals in the form of tatrates dissolved in water. (The endovenous administration provides the highest bio-availability of the products). The results are shown in Table 3, together with the comparison ones obtained employing physostigmine.

Table 3

| Acute toxicity . $LD_{50}$ values | | |
|---|---|---|
| Product | $LD_{50}$ in mg/kg | |
| | endovenous | oral |
| Example 2 | >50<br><100 | >100<br><150 |
| Example 3 | >50<br><100 | >178 |
| Example 4 | >50<br><100 | >200 |
| Example 6<br>Physostigmine | <50<br>0.58 | ca.70<br>2.2 |

As can be seen from the results reported in Table 3, the products of the invention show a low toxicity when administered orally or endovenously to the test animals.

In particular, the compounds of Examples 3 and 4 have an acute toxicity which is approximately 100 times lower than the one of physostigmine.

Some examples of the preparation of physostigmine derivatives of formula (I) are reported below.

Example 1

Preparation of N-methyl,N-prophyl-carbamyl chloride

In a two neck round bottom flask are introduced 700 mg (2.3 mmols) bis-(trichloromethyl)-carbonate dissolved in 10 ml anhydrous benzene. In the flask, cooled in a iced water bath and in a nitrogen atmosphere, are introduced 0.5 ml pyridine and, dropwise with a syringe, 570 mg ($d_r$ = 0.7; 7,8 mmols) N-methyl-N-propylamine dissolved in 10 ml anhydrous benzene. The reaction mixture is kept under nitrogen for 48 hrs. The N-methyl-N-propyl-carbamylchloride is extracted from the crude reaction solid with carbon tetrachloride. After evaporation of the solvent 900 mg (6.64 mmols) product are obtained in a 85% yield.

Example 2

Preparation of 1,2,3,3a,8,8a-hexaydro-1,3a,8-trimethylpyrrol(2,3-b)indol-5-ol(3aS-cis)-N-methyl,N-propylcarbamate (ester)

2.5 g (9 mmols) physostigmine and 800 mg (15 mmols) sodium methoxide are introduced in a 250 ml flask, in which the pressure is reduced to a controlled value of between 1,300 and 2,600 Pascal. 20 ml benzene are added and, in about 2 hrs in small volumes, approximately 80 ml ethyl alcohol. The mixture is kept under stirring at room temperature. After removing the ethanol, approximately 100 ml benzene are added at room temperature under stirring. To this solution 20 ml of a benzene solution containing 9 mmols N-methyl-N-propyl-carbamyl chloride (prepared as described in Example 1) are added. The reaction mixture is left standing for 3 hrs to allow for the carbamate formation.

Then, an equal volume of petroleum ether is added, the vacuum is removed and the solution is kept at room temperature for a time of between 12 and 16 hrs.

The organic solution is washed several times with cold water and is then extracted with an approximately 0.1 M malic acid solution. The organic solution is discharged, while the water solution is washed with petroleum ehter. The acidic water solution is saturated with sodium bicarbonate and extracted again with petroleum ether contaning 5% benzene.

The organic layer is washed with water and dried on magnesium sulphate. Finally, the solvent is removed under reduced pressure and, if necessary, the residue is chromatographed in a silica-gel column (eluent chloroform: methanol 9:1) - 1.8 g of oil are obtained which is dissolved in ether adding then a 1:1 petroleum ether: heptane mixture. From the solution a powder separates slowly having a m.p. of 85-88°C.

| Elemental analysis (for $C_{18}H_{37}N_3O_2$): | | | |
|---|---|---|---|
| calculated<br>found | C = 68.14%,<br>C = 68.00%, | H = 8.52%<br>H = 8.59% | N = 13.25%<br>N = 13.10% |

The compound structure was confirmed by UV, IR and NMR analysis.

Example 3

Preparation of 1,2,3,3a,8,8a-hexahydro-1,3a,8-trimethylpyrrol(2,3-b)indol-5-ol(3aS-cis)-N-methyl,N-pentylcarbamate (ester)

Operating as in Example 2, starting from 1.0 g physostigmine (3.65 mmols) and 260 g sodium methoxide (5 mmols), 750 mg (5 mmols) N-methyl-N-pentylcarbamylchloride (prepared as described in Example 1 starting from bis(trichloromethyl)-carbonate and N-methyl-N-pentylamine) are added. 0.5 g oil are obtained.

| Elemental analysis (for $C_{20}H_{31}N_3O_2$): | | | |
|---|---|---|---|
| Calculated<br>Found | C = 69.56%,<br>C = 69.35%, | H = 9.15%,<br>H = 9.15%, | N = 12.10%<br>N = 12.10% |

The structure of the compound was confirmed by UV, IR and NMR analysis.

Example 4

Preparation of 1,2,3,3a,8,8a-hexahydro-1,3a,8-trimethylpyrrol(2,3-b)indol-5-ol (3aS-cis) N-methyl,N-heptylcarbamate (ester)

One operates as in Example 2, employing 1.5 g physostigmine (5.45 mmols) and 400 mg sodium methoxide (7.4 mmols). 950 mg (5 mmols) N-methyl-N-heptylcarbamylchloride, are added, prepared as described in Example 1, starting from bis(trichlormethyl) carbonate and N-methyl-N-heptylamine, this last being prepared as in the successive Example 7.

1.2 g oil are obtained.

| Elemental analysis (for $C_{22}H_{35}N_3O_2$): | | | |
|---|---|---|---|
| Calculated:<br>Found: | C = 70.78%,<br>C = 70.50%, | H = 9.38%,<br>H = 9.48% | N = 11.26%<br>N = 11.12% |

The structure of the compound was confirmed by UV, IR and NMR analysis.

Example 5

Preparation of 1,2,3,3a,8,8a-hexahydro-1,3a,8-trimethylpyrrol (2,3-b)indol-5-ol (3aS-cis) N-methyl,N-nonylcarbamate (ester)

One operates as in Example 2, employing 1.0 g physostigmine (3.65 mmols) and 400 mg sodium

methoxide (7.4 mmols). 880 mg N-methyl-N-nonyl-carbamyl chloride (prepared as described in Example 1 starting from bis-(trichloromethyl)-carbonate and N-methyl-N-nonylamine).

0.6 g of oil are obtained.

| Elemental analysis (for $C_{24}H_{39}N_3O_2$) | | | |
|---|---|---|---|
| Calculated: | C = 71.82%, | H = 9.73%, | N = 10.47% |
| Found: | C = 71.60%, | H = 9.85%, | N = 10.27% |

The structure of the compound was confirmed by UV, IR and NMR analysis.

Example 6

Preparation of 1,2,3,3a,8,8a-hexahydro-1,3a,8-trimethylpyrrol.(2,3-b)indol-5-ol (3aS-cis) N-methyl,N-benzyl carbamate (ester)

Operating as in Example 2, employing 1.5 g physostigmine (5.45 mmols) and 360 mg sodium methoxide (6.7 mmols) 1.0 g N-methyl-N-benzylcarbamylchloride, prepared as described in Example 1 starting from bis(trichloromethyl) carbonate and N-metyl-N-benzylamine, are added.

1.3 g oil are obtained.

| Elemental analysis: | | | |
|---|---|---|---|
| Calculated: | C = 72.33%, | H = 7.40%, | N = 11.51% |
| Found: | C = 72.09%, | H = 7.50%, | N = 11.22% |

The structure of the compund was confirmed by UV, IR and NMR analysis.

Example 7

Preparation of N-methyl,N-heptyl-amine

50 ml of a commercial 40% water solution of methylamine are heated and the vapors bubbled into a three-neck round bottom flask (provided with magnetic stirrer, in a nitrogen atmosphere and cooled in a water-ice bath) containing 100 ml ethanol. After one hour, there is added, dropwise through a funnel, 1 ml jodoheptane dissolved in 10 ml benzene. The water-ice bath is removed and the flask is heated on mild reflux for about 3 hours. The reaction mixture is then evaporated to dryness, taken up with $CHCl_3$ and agitated with a 10% NaOH water solution.

The aqueous layer is separated and discarded.

The chloroform solution is acidified and the chloroform layer is separated and discarded. The acidic water solution is made alkaline with NaOH and extracted again with chloroform. After drying of chloroform solution on anhydrous magnesium sulphate, and evaporation of the solvent, the secondary amine is spectroscopically identified (NMR) as N-methyl,N-heptylamine. The physostigmine derivatives of formula (I) and their salts with pharmaceutically acceptable acids are particularly useful for the preparation of pharmaceutical compositions for the treatment of cerebral affections against which the benefical effects of physostigmine are known, such as e.g. glaucoma, myastenia gravis, Alzheimer's disease and also organophosphorus compounds poisoning.

The high activity of the compounds according to the invention, their good stability and reduced toxicity clearly allow to prepare compositions of a high clinical interest.

Effective amounts of physostigmine derivatives of formula (I) according to the invention, as such or in the form of salts, may be administered to patients orally, or introperitoneally, or endovenously, employing all excipients and vehicles normally employed in the pharmaceutical praxis.

Typically, the oral formulations are prepared as pills, tablets, capsules, syrups, while the ones for intraperitoneal, endomuscular or endovenous use are in the form of solutions or suspensions in sterile diluents, such as physiological solution, saline solutions or solvents normally employed in the pharmaceutical praxis.

The amount of active component in such formulations may vary within broad limits, from 0.5% to 70%

by wt. of active compound, and adequate for an effective dosage of the active component.

The preferred compositions have an active compound content comprised between 1 and 300 mg per administration unit.

Given the high activity of the physostigmine derivatives of the invention and their protracted action, it is of particular interest to formulate pharmaceutical compositions to be used per os once a day, with active principle of between 50 and 200 mg per unit.

## Claims

1. Physostigmine derivatives having the general formula:

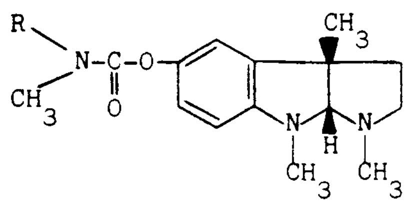

(I)

wherein R is a linear or branched alkyl with three to nine C atoms, or benzyl, and their salts with pharmaceutically acceptable acids.

2. Physostigmine derivatives according to claim 1, the salts of which are formed with citric-, tartaric- or maleic acid.

3. Process for preparing physostigmine derivatives having the general formula

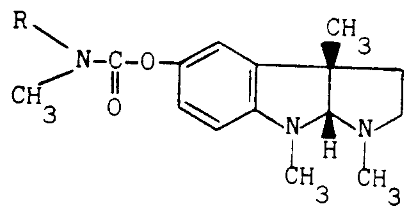

(I)

in which R is a linear or branched alkyl with three to nine carbon atoms, or benzyl, characterized in that

a) physostigmine is reacted, at room temperature and in a nitrogen atmosphere, with sodium methoxide and an aliphatic alcohol having 1 to 3 carbon atoms, in a low polarity organic solvent until it is transformed into eseroline (II)

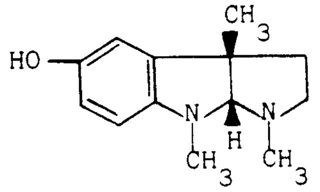

(II)

b) the alcohol is distilled off and the eseroline solution is reacted with a solution, in the same organic solvent, of a carbamyl chloride of general formula R-N($CH_3$)-CO-Cl (III) wherein R is a linear or branched alkyl with three to nine C atoms, or benzyl, to obtain the corresponding physostigmine derivative (I) in non-salified form;

c) at the end of the reaction, the mixture is treated with a solution in an organic solvent of the acid with which one desires to form the physostigmine derivative salt, optionally it is washed with cold

water and the derivative as such is purified by extraction with apolar solvent.

4. Process according to claim 3, for preparing physostigmine derivative in which said low polarity organic solvent is selected from the groups consisting of dioxane, ethylene glycol, benzene, ethyl ether, diisopropyl ehter, toluene, xylene, petroleum ether.

5. Process according to claim 3, for preparing physostigmine derivatives, in which said carbamyl chloride solution is added slowly to the eseroline solution in an amount such as to have a ratio of between 1:1 and 1:1.4 between the two reagents.

6. Process according to claim 3, for preparing physostigmine derivatives, in which the salt form of the physostigmine derivative of formula (I) is prepared by dissolving said derivative in an organic solvent selected form the group consisting of ethyl ether, ethanol, methanol, iso-propanol, diisopropyl ether and their mixtures, and the thus obtained solution is reacted with the appropriate acid dissolved in the same solvent, the reagent concentrations in the solvent being comprised between 50 and 250 ml solvent for g of physostigmine derivative and 50 and 350 ml solvent per g of acid.

7. Pharmaceutical compositions containing therapeutically effective amounts of the physostigmine derivatives according to claim 1, together with other pharmaceutically acceptable constituents.

8. The use of physostigmine derivatives according to claim 1 for preparing pharmaceutical compositions useful for the treatment of glaucoma, myastenia gravis, Alzheimer's disease and poisoning from organophosphorous compounds.

European Patent Office

# EUROPEAN SEARCH REPORT

Application Number

EP 91 11 9503

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 253 372 (HOECHST-ROUSSEL)<br>* page 1, paragraph 2 - page 1, paragraph 3; claim 1 *<br>--- | 1,7 | C07D487/04<br>A61K31/40<br>//(C07D487/04, 209:00,209:00) |
| X | ACTA MEDICA ROMANA<br>vol. 25, 1987, ROME, ITALY<br>pages 438 - 446;<br>M. MARTA ET AL.: 'Cholinergic drugs in senile dementia of Alzheimerαs type'<br>* complete article *<br>--- | 1,7 | |
| X | EP-A-0 154 864 (CONSIGLIO NAZIONALE DELLE RICERCHE)<br>* claims 1,5 *<br>----- | 1,7 | |
| | | | TECHNICAL FIELDS SEARCHED (Int. Cl.5 ) |
| | | | C07D<br>A61K |

The present search report has been drawn up for all claims

| Place of search | Date of completion of the search | Examiner |
|---|---|---|
| THE HAGUE | 17 MARCH 1992 | ALFARO FAUS I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document